(19) Europäisches Patentamt
European Patent Office
Office européen des brevets

(11) Veröffentlichungsnummer: **0 185 221**
**B1**

(12) # EUROPÄISCHE PATENTSCHRIFT

(45) Veröffentlichungstag der Patentschrift: **26.09.90**

(51) Int. Cl.[5]: **C07D 277/20**

(21) Anmeldenummer: **85114900.5**

(22) Anmeldetag: **25.11.85**

(54) **Verfahren zur Herstellung von Aminothiazolessigsäurederivaten.**

(30) Priorität: **19.12.84 CH 6008/84**

(43) Veröffentlichungstag der Anmeldung: **25.06.86 Patentblatt 86/26**

(45) Bekanntmachung des Hinweises auf die Patenterteilung: **26.09.90 Patentblatt 90/39**

(84) Benannte Vertragsstaaten: **AT BE CH DE FR GB IT LI LU NL SE**

(56) Entgegenhaltungen:
**EP-A- 0 096 296**
**EP-A- 0 096 297**

(73) Patentinhaber: **F. HOFFMANN-LA ROCHE AG, Postfach 3255, CH-4002 Basel(CH)**

(72) Erfinder: **Hebeisen, Paul, Dr., Klusweg 51, Ch-4153 Reinach(CH)**

(74) Vertreter: **Notegen, Eric-André et al, Grenzacherstrasse 124 Postfach 3255, CH-4002 Basel(CH)**

EP 0 185 221 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Verbindungen der allgemeinen Formel

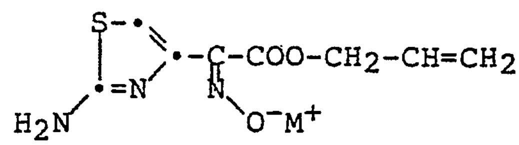 I

worin M ein Alkalimetallatom bedeutet,
das dadurch gekennzeichnet ist, dass man eine Verbindung der allgemeinen Formel

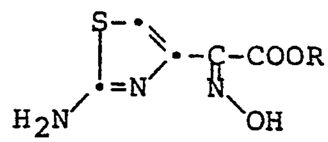 II

worin R niederes Alkyl bedeutet,
unter im wesentlichen wasserfreien Bedingungen und in einem im wesentlichen aus Allylalkohol bestehenden Lösungsmittel mit einer Verbindung der allgemeinen Formel

$CH_2=CH\text{-}CH_2\text{-}O^-M^+$ III

worin M obige Bedeutung besitzt,
umsetzt.

Die vorliegende Erfindung betrifft ferner ein Verfahren zur Herstellung von Verbindungen der allgemeinen Formel

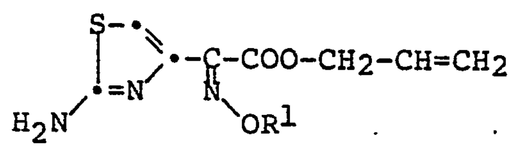 IV

worin $R^1$ niederes Alkyl, niederes Alkanoyl, niederes Alkenyl oder die Gruppe $-CH_2COOR^2$ oder $-C(CH_3)_2COOR^2$ und $R^2$ eine leicht abspaltbare Gruppe bedeuten,
das dadurch gekennzeichnet ist, dass man eine erfindungsgemäss erhaltene Verbindung der Formel I mit einer Verbindung der allgemeinen Formel

$R^1\text{-}X$ V

worin X eine Abgangsgruppe bedeutet, und $R^1$ obige Bedeutung besitzt,
umsetzt, sowie die Anwendung dieser Verfahren zur Herstellung von antimikrobiell wirksamen mono-β-Lactam-, Cephalosporin- und Penicillinderivaten, deren Aminogruppe am β-Lactamring mit einer Gruppe der allgemeinen Formel

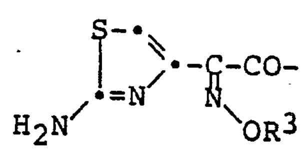 (Q)

worin $R^3$ Wasserstoff, niederes Alkyl, niederes Alkenyl oder die Gruppe $-CH_2COOR^4$ oder $-C(CH_3)_2COOR^4$ und $R^4$ Wasserstoff oder eine leicht abhydrolysierbare Gruppe bedeuten,
substituiert ist.

Der Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl und s-Butyl. Der Ausdruck "Alkenyl" bezeichnet geradkettige oder verzweigte, ungesättigte Kohlenwasserstoffreste, wie Allyl, 2-Butenyl und 3-Butenyl. Der Ausdruck "Alkanoyl" bezeichnet geradkettige oder verzweigte Fettsäurereste, wie Acetyl, Propanoyl und Isobutyryl.

Der Ausdruck "leicht abspaltbare Gruppe" bezeichnet vorzugsweise eine gegebenenfalls am Phenylring durch Halogen, niederes Alkoxy oder Nitro substituierte Benzylgruppe, wie Benzyl, p-Nitrobenzyl, p-Methoxybenzyl, 2,4- oder 3,4-Dimethoxybenzyl und p-Chlorbenzyl, t-Alkylgruppen, wie t-Butyl, in der 2-Stellung halogenierte niedere Alkylgruppen, wie 2,2,2-Trichloräthyl, 2-Bromäthyl und 2-Jodäthyl, in der 2-Stellung silylierte niedere Alkylgruppen, wie 2-Trimethylsilyläthyl, und leicht abhydrolysierbare, vorzugsweise unter physiologischen Bedingungen abhydrolysierbare Gruppen, insbesondere niedere Alkanoyloxy-niedere Alkylgruppen, wie die Acetoxymethyl-, Pivaloyloxymethyl-, 1-Acetoxyäthyl- und die 1-Pivaloyloxyäthylgruppe, und niedere Alkoxycarbonyloxy-niedere Alkylgruppen, wie die Methoxycarbonyloxymethyl-, 1-Aethoxycarbonyloxyäthyl- und die 1-Isopropoxycarbonyloxyäthylgruppe.

Der Ausdruck "Abgangsgruppe" bezeichnet herkömmliche Abgangsgruppen, wie Sulfonyloxygruppen und Halogenatome. Geeignete Sulfonyloxygruppen sind beispielsweise niedere Alkylsulfonyloxygruppen, wie die Methansulfonyloxygruppe, und Arylsulfonyloxygruppen, wie die p-Toluolsulfonyloxygruppe. Geeignete Halogenatome sind beispielsweise die Chlor-, Brom- und Jodatome.

Die erfindungsgemässe Umsetzung einer Verbindung der Formel II mit einem Alkalimetallallylat der Formel III kann in einem weiten Temperaturbereich, beispielsweise in einem Bereich von etwa 0°C bis zur Siedetemperatur des Reaktionsgemisches durchgeführt werden. Zweckmässigerweise arbeitet man jedoch bei Raumtemperatur. Als Ausgangsstoff der Formel II verwendet man vorzugsweise den entsprechenden Aethylester, eine im Handel erhältliche Verbindung. Als Ausgangsstoff der Formel III verwendet man vorzugsweise Kaliumallylat.

Die Alkalimetallallylate der Formel III können durch Umsetzen von Allylalkohol mit Alkalimetallen, Alkalimetallhydriden, wie Natriumhydrid, Alkalimetallalkylen, wie n-Butyllithium, oder Alkalimetallhydroxiden, wie Kaliumhydroxid, nach an sich bekannten Methoden hergestellt werden.

Zur Durchführung der Umsetzung einer erfindungsgemäss erhaltenen Verbindung der Formel I mit einer Verbindung der Formel V wird die Verbindung der Formel I zweckmässigerweise in einem inerten organischen Lösungsmittel suspendiert oder gelöst. Geeignete Lösungsmittel sind beispielsweise Kohlenwasserstoffe, wie Pentan, Hexan, Cyclohexan, Petroläther, Benzol, Toluol und Xylol, Aether, wie Tetrahydrofuran, Dioxan, Aethylenglykoldimethyläther, Diäthyläther und t-Butylmethyläther, N,N-Dimethylformamid, Aceton, Essigester, Acetonitril und Dimethylsulfoxid. Die Reaktion kann in einem weiten Temperaturbereich, beispielsweise in einem Bereich von etwa 0°C bis zur Siedetemperatur der Reaktionsmischung durchgeführt werden. Als Ausgangsstoff der Formel V verwendet man vorzugsweise ein entsprechendes Halogenid.

Wie bereits erwähnt, können die erfindungsgemässen Verfahren zur Herstellung von antimikrobiell wirksamen mono-β-Lactam-, Cephalosporin- und Penicillinderivaten, deren Aminogruppe am β-Lactamring mit einer Gruppe der Formel Q substituiert ist, angewendet werden. Die Verbindungen der Formel IV gehören einer an sich bekannten Stoffklasse an und können in Analogie zu den bekannten Vertretern dieser Stoffklasse in die gewünschten, antimikrobiell wirksamen mono-β-Lactame, Cephalosporine und Penicilline übergeführt werden.

Die nachfolgenden Beispiele, welche die vorliegende Erfindung erläutern sollen, enthalten auch detaillierte Angaben betreffend die Anwendung der erfindungsgemässen Verfahren zur Herstellung der vorerwähnten antimikrobiell wirksamen Endprodukte.

Beispiel 1

a) Man löst 33,7 g (600 mM) Kaliumhydroxid in 200 ml Allylalkohol, gibt 300 ml Toluol dazu, filtriert die leicht trübe Lösung und engt bei 30°C im Vakuum ein (Entfernung von Wasser). Der ölige Rückstand wird nochmals in 300 ml Toluol aufgenommen und die Lösung wird erneut im Vakuum eingeengt. Das erhaltene Kaliumallylat wird in 900 ml Allylalkohol gelöst, worauf man mit 64,5 g (300 mM) 2-(2-Amino-4-thiazolyl) -2-syn-hydroxyimino-essigsäure-äthylester versetzt und 2 Tage bei Raumtemperatur rührt. Das auskristallisierte Material wird abgenutscht, sukzessive mit Allylalkohol, Essigester und Aether gewaschen und im Vakuum bei 40°C getrocknet. Man erhält das Kaliumsalz des 2-(2-Amino-4-thiazolyl) -2-syn-hydroxyimino-essigsäure-allylesters vom Smp. >250°C.

b) Man suspendiert 76,2 g (287,2 mM) des Kaliumsalzes des 2-(2-Amino-4-thiazolyl) -2-syn-hydroxyimino-essigsäure-allylesters in 1,4 l Tetrahydrofuran, kühlt auf 0°C ab und versetzt unter Rühren mit 20,4 ml (287,2 mM) Acetylchlorid. Nach 1 Stunde wird auf ein kleines Volumen eingeengt und mit 3 l Essigester versetzt. Die erhaltene Lösung wird dreimal mit Wasser gewaschen, und die wässrigen Extrakte werden mit 1 l Essigester ausgeschüttelt. Die dunkel gefärbte organische Phase wird mit Tierkohle behandelt, über Magnesiumsulfat getrocknet und im Vakuum eingedampft. Man nimmt den Rückstand in 1 l Methylenchlorid auf, filtriert, um ungelöstes Material zu entfernen, versetzt dann mit 2 l Tetrachlorkohlenstoff und engt auf ein kleines Volumen ein. Das auskristallisierte Material wird abgenutscht, mit Tetrachlorkohlenstoff und Petroläther gewaschen und im Vakuum bei 40°C getrocknet. Man erhält 2-(2-Amino-4-thiazolyl) -2-syn-acetoxyimino-essigsäure-allylester vom Smp. 144-147°C.

c) Man löst 54 g (200 mM) 2-(2-Amino-4-thiazolyl) -2-syn-acetoxyimino-essigsäure-allylester unter Stickstoff in 1,4 l Acetonitril, kühlt auf 0°C ab und versetzt sukzessive mit 448 mg (2 mM) Palladium(II)acetat und 1,72 ml (10 mM) Triäthylphosphit. Nach 5 Minuten gibt man 23,2 ml (220 mM) N-Methylpyrrolidin dazu und rührt über Nacht und unter Stickstoff bei 0°C, wobei das N-Allyl-N-methylpyrrolidiniumsalz der 2-(2-Amino-4-thiazolyl)-2-syn-acetoxyimino-essigsäure auskristallisiert.

d) Anschliessend werden 28 ml (248 mM) N-Methylmorpholin und nach 30 Minuten 74 g (220 mM) 2,2-Dithio-bis-benzothiazol dazugegeben. Innert 4 Stunden wird dann unter Rühren eine Lösung von 42 ml (245 mM) Triäthylphosphit in 200 ml Acetonitril bei 0°C dazugetropft. Anschliessend wird noch während 30 Minuten gerührt. Das auskristallisierte Material wird abgenutscht, zuerst mit Acetonitril und dann mit Aether gewaschen und im Vakuum bei 30°C getrocknet. Man erhält den 2-(2-Amino-4-thiazolyl) -2-syn-acetoxyimino-essigsäure-2-benzthiazolyl-thioester vom Smp. 170-172°C.

e) Man suspendiert 29,6 g (80 mM) 7-Amino-3-{[(2,5-dihydro-6-hydroxy-2-methyl-5-oxo-as -3-triazinyl)thio]methyl}- -3-cephem-4-carbonsäure in 560 ml N,N-Dimethylformamid, versetzt mit 24,8 ml (176 mM) Triäthylamin, kühlt auf 0°C ab und gibt 80 ml Wasser dazu. Zur erhaltenen klaren Lösung werden 38 g (100 mM) 2-(2-Amino-4-thiazolyl) -2-syn-acetoxyimino-essigsäure-2-benzthiazolyl-thioester in fester Form zugegeben, und das Reaktionsgemisch wird 3 Stunden bei 0°C gerührt. Die dunkle Lösung wird filtriert, wobei man zweimal mit je 40 ml N,N-Dimethylformamid wäscht. Man kühlt die Lösung auf 0°C ab, versetzt mit 96 ml (192 mM) einer 2N-Lösung von 2-Aethylcapronsaurem Natrium in Essigester, gibt innert 30 Minuten bei 0°C und unter Rühren 1080 ml Aceton dazu, nutscht das ausgefallene Material ab, wäscht es zuerst mit 500 ml eines Gemisches aus Aceton und N,N-Dimethylformamid im Verhältnis 3:2 und dann mit 500 ml Aceton und trocknet es im Vakuum bei Raumtemperatur. Man erhält das Dinatriumsalz der 7-[2-(2-Amino-4-thiazolyl)-2-syn-acetoxyiminoacetamido] -3-{[2,5-dihydro-6-hydroxy-2-methyl-5-oxo-as -3-triazinyl)thio]methyl}-3-cephem-4- carbonsäure, das gemäss HPLC einen Reinheitsgrad von 96,6% aufweist.

f) Man löst 48 g (76,6 mM) des Dinatriumsalzes der 7-[2-(2- Amino-4-thiazolyl)-2-syn-acetoxyiminoacetamido] -3-{[(2,5-dihydro-6-hydroxy-2-methyl-5-oxo-as -3-triazinyl)- thio]methyl}-3-cephem-4-carbonsäure in einer auf 25°C abgekühlten Lösung von 520 ml Methanol und 400 ml Wasser. Der pH der Lösung wird mittels 1N-Natronlauge auf 8 gestellt und während der ganzen Reaktionsdauer bei diesem pH gehalten, wobei der Verbrauch an Natronlauge sehr gering ist. Nach beendeter Reaktion (der Reaktionsablauf wird mittels HPLC verfolgt) wird der pH mit 1N-Salzsäure auf 7 gestellt und die dunkel gefärbte Lösung wird während 20 Minuten mit 10 g Aktivkohle gerührt. Anschliessend wird genutscht, wobei man den Rückstand noch mit einem Gemisch aus 52 ml Methanol und 40 ml Wasser wäscht. Die erhaltene gelbe Lösung wird unter Rühren mit 2,4 l Alkohol versetzt, worauf man auf 0°C abkühlt, das ausgefallene Material abnutscht, zuerst mit 350 ml eines Gemisches aus Alkohol und Wasser (6:1) und dann mit 450 ml Alkohol wäscht und anschliessend bei 1600 Pa und Raumtemperatur und dann im Hochvakuum trocknet. Man erhält 34,7 g (77,5%) des Dinatriumsalzes der 7-[2-(2-Amino-4-thiazoly)-2-syn-hydroxyiminoacetamido] -3-{[(2,5-dihydro-6- hydroxy-2-methyl-5-oxo-as -3-triazinyl)thio]methyl}-3-cephem-4-carbonsäure, das noch 5,9% Aethanol und 4,9% Wasser enthält.

Mikroanalyse: [Berechnet für $C_{17}H_{14}N_8Na_2O_7S_3$
(584,51) + 5,9% Aethanol + 4,9% Wasser]:
Berechnet: C 34,24; H 3,48; N 17,10
Gefunden: C 33,79; H 3,20; N 17,24

Beispiel 2

a) Zu 200 ml Allylalkohol werden bei 2°C portionenweise 4,30 g Natriumhydrid (99-proz.) zugegeben. Nach Beendigung der Gasentwicklung werden 40,0 g 2-(2-Amino-4-thiazolyl) -2-syn-hydroxyimino-essigsäure-äthylester in einer Portion zugegeben, und die Mischung wird 2 Stunden bei Raumtemperatur gerührt. Darauf werden unter Vakuum 40 ml Lösungsmittel abdestilliert, und der Rest wird bei Raumtemperatur über Nacht gerührt. Das Produkt wird abfiltriert, mit Essigester und t-Butylmethyläther gewaschen und im Hochvakuum zur Gewichtskonstanz getrocknet. Man erhält das Natriumsalz des 2-(2-Amino-4-thiazolyl) -2-syn-hydroxyimino-essigsäure-allylesters.

b) Zu 240 ml Allylalkohol tropft man bei -78°C 62 ml n-Butyllithium (1,6M in Hexan), lässt auf Raumtemperatur erwärmen und entfernt 100 ml Lösungsmittel im Vakuum. Man gibt dann 21,5 g 2-(2-Amino-4-thiazolyl) -2-syn-hydroxyimino-essigsäure-äthylester dazu und rührt das Reaktionsgemisch über Nacht. Das Produkt wird abfiltriert, mit Essigester und t-Butylmethyläther gewaschen und im Hochvakuum zur Gewichtskonstanz getrocknet. Man erhält das Lithiumsalz des 2-(2-Amino-4-thiazolyl) -2-syn-hydroxyimino-essigsäure-allylesters vom Smp. >260°C.

c) Man löst unter Rühren 337 g Kaliumhydroxid in 2 l Allylalkohol, vesetzt mit 3 l Toluol und dampft die Mischung im Vakuum (2000 Pa) ein. Man versetzt nochmals mit 3 l Toluol und dampft erneut ein. Der Rückstand wird in 9 l Allylalkohol aufgenommen, worauf man mit 645 g 2-(2-Amino-4-thiazolyl) -2-syn-hydroxyimino-essigsäure-äthylester versetzt und 45 Stunden bei Raumtemperatur rührt. Das Produkt wird abfiltriert, mit 1 l Allylalkohol, 8 l Essigester und 2,5 l t-Butylmethyläther gewaschen und im Hochvakuum bei 40°C zur Gewichtskonstanz getrocknet. Man erhält das Kaliumsalz des 2-(2-Amino-4-thiazolyl) -2-syn-hydroxyimino-essigsäure-allylesters.

$^1$HNMR-Spektrum in $(CD_3)_2SO$: Jeweils Signale bei 9,20 (s breit, 2H); 5.96 (s, 1H); 6,3-5,0 (m, 3H); 4,66 (d, J=6,5 Hz, 2H) ppm.

Beispiel 3

4,60 g des Lithiumsalzes des 2-(2-Amino-4-thiazolyl)-2-syn-hydroxyimino-essigsäure-allylesters werden in 20 ml trockenem N,N-Dimethylformamid gelöst, worauf man tropfenweise mit 3,4 ml frisch destilliertem Allylbromid versetzt. Die Temperatur steigt dabei auf 50°C. Nach 15 Minuten versetzt man mit gesättigter Ammoniumschloridlösung und extrahiert mit Essigester. Die vereinigten organischen Phasen werden zweimal mit Wasser und mit gesättigter Kochsalzlösung gewaschen und über Magnesiumsulfat getrocknet. Der nach Abdampfen des Lösungsmittels erhaltene Rückstand wird aus t-Butylmethyläther umkristallisiert. Man erhält 2-(2-Amino-4-thiazolyl) -2-syn-allyloxyimino-essigsäure-allylester vom Smp. 106-107°C.

Beispiel 4

a) Zu 360 ml einer 0,5 Mol Kaliumallylat enthaltenden Lösung von Kaliumallylat in Allylalkohol gibt man 82 g (0,38 Mol) 2-(2-Amino-4-thiazolyl) -2-syn-hydroxyimino-essigsäure-äthylester. Die Suspension wird 20 Stunden lang bei Raumtemperatur unter Argon gerührt, worauf man das ausgefallene Material abnutscht, mit Essigester und Aether wäscht und bei 50°C im Vakuum (1600 Pa) trocknet. Man erhält das Kaliumsalz des 2-(2-Amino-4-thiazolyl) -2-syn-hydroxyimino-essigsäure-allylesters.

b) Man suspendiert 39,5 g des obigen Kaliumsalzes bei Raumtemperatur in 450 ml absolutem Aceton, versetzt mit 5 g Kaliumjodid und 26,9 g Chloressigsäure-t-butylester und erhitzt anschliessend unter Argon während 4 Stunden unter Rückfluss. Danach giesst man das Gemisch auf 750 ml Essigester und 750 ml Wasser, rührt gut durch, trennt die Phasen und wäscht die wässrige Phase noch mit 400 ml Essigester. Die vereinigten Essigesterphasen werden mit gesättigter Kochsalzlösung gewaschen, über Natriumsulfat getrocknet und eingedampft. Der Rückstand wird aus 540 ml Toluol umkristallisiert, wobei man 2-(2-Amino-4-thiazolyl) -2-syn-[[(t-butoxycarbonyl)methoxy]imino] -essigsäure- allylester vom Smp. 136-138°C erhält.

c) Man suspendiert 21,5 g 2-(2-Amino-4-thiazolyl) -2-syn- [[(t-butoxycarbonyl)methoxy]imino] -essigsäure-allylester in 150 ml absolutem Acetonitril, gibt dann unter Rühren und unter Argon nacheinander 120 mg Palladium(II)acetat, 0,5 ml Triäthylphosphit und 5,3 ml N-Methylpyrrolidin dazu und rührt etwa 1,5 Stunden bei Raumtemperatur, wobei das N-Allyl-N-methylpyrrolidinium-Salz der 2-(2-Amino-4-thiazolyl) -2-syn-[[(t-butoxycarbonyl)methoxy]imino]-essigsäure auskristallisiert. Anschliessend versetzt man die Suspension mit 6 ml N-Methylmorpholin und 20 g Dithio-bis-2-benzthiazol, kühlt auf -5°C bis 0°C ab und tropft dann während 2 Stunden eine Lösung von 12 ml Triäthylphosphit in 30 ml absolutem Acetonitril dazu. 1 Stunde nach Beendigung der Zugabe wird auf -10°C abgekühlt, und der kristalline Thiolester wird abgenutscht, nacheinander mit 35 ml Acetonitril und 35 ml Aether gewaschen und bei 60°C im Vakuum (1600 Pa) getrocknet. Man erhält 2-(2-Amino-4-thiazolyl) -2-syn-[[(t- butoxycarbonyl)methoxy]-imino] -essigsäure-2-benzthiazolyl- thioester vom Smp. 142-144°C.

d) 71,8 g (0,3 Mol) (3S,4S)-3-Amino-4-carbamoyloxymethyl -2-oxo-1-azetidinsulfonsäure werden in 1,5 l Methylenchlorid dispergiert, worauf man mit 45,6 g (0,45 Mol) Triäthylamin und 148,6 g (0,33 Mol) 2-(2-Amino-4-thiazolyl) -2-syn-[[(t- butoxycarbonyl)methoxy]imino]essigsäure -2-benzthiazolylthioester unter Rühren versetzt. Das Reaktionsgemisch wird 5 Stunden bei Raumtemperatur gerührt. Anschliessend gibt man 1,5 l Wasser dazu, trennt die wässrige Phase ab, extrahiert sie zweimal mit je 250 ml Methylenchlorid und säuert sie durch Zugabe von 850 ml 37-proz. wässriger Salzsäure an. Nach Rühren bei Raumtemperatur während 2 Stunden wird die erhaltene Suspension auf 0°C abgekühlt und weitere 0,5 Stunden gerührt. Der Niederschlag wird abfiltriert, nacheinander mit 1000 ml kaltem Wasser, 1000 ml Methanol und 1000 ml Aether gewaschen und 12 Stunden bei 40°C im Vakuum (1600 Pa) getrocknet. Man erhält (3S,4S)-3-{(Z)-2-(2-Amino-4-thiazolyl) 2-[(carboxymethoxy)imino]acetamido} -4-carbamoyloxy- methyl-2-oxo-1-azetidinsulfonsäure vom Smp. 207°C.

Beispiel 5

Man suspendiert 11,66 g des Lithiumsalzes des 2-(2-Amino-4-thiazolyl) -2-syn-hydroxyimino-essigsäure- allylesters bei Raumtemperatur in 100 ml trockenem Tetrahydrofuran, versetzt mit 6,2 ml Pivaloylchlorid, rührt während 45 Minuten, dampft das Lösungsmittel ab und verteilt den Rückstand zwischen Essigester und Wasser. Die wässrige Phase wird noch zweimal mit Essigester extrahiert. Die vereinigten organischen Phasen werden zweimal mit gesättigter Bicarbonatlösung und je einmal mit Wasser und gesättigter Kochsalzlösung gewaschen, über Magnesiumsulfat getrocknet und eingedampft. Der Rückstand wird aus t-Butylmethyläther umkristallisiert, und man erhält 2-(2-Amino-4-thiazolyl) -2-syn-pivaloyloxyimino- essigsäure-allylester vom Smp. 141-143°C.

**Patentansprüche**

1. Verfahren zur Herstellung von Verbindungen der allgemeinen Formel

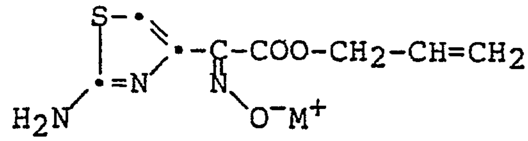 I

worin M ein Alkalimetallatom bedeutet, dadurch gekennzeichnet, dass man eine Verbindung der allgemeinen Formel

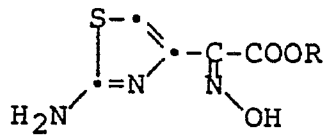 II

worin R niederes Alkyl mit höchstens 7 Kohlenstoffatomen bedeutet,
unter im wesentlichen wasserfreien Bedingungen und in einem im wesentlichen aus Allylalkohol bestehenden Lösungsmittel mit einer Verbindung der allgemeinen Formel

$$CH_2=CH-CH_2-O^-M^+ \qquad III$$

worin M obige Bedeutung besitzt, umsetzt.

2. Verfahren nach Anspruch 1, dadurch gekennzeichnet, dass M ein Kaliumatom bedeutet.

3. Verfahren nach Anspruch oder 2, dadurch gekennzeichnet, dass R Aethyl bedeutet.

4. Verfahren zur Herstellung von Verbindungen der allgemeinen Formel

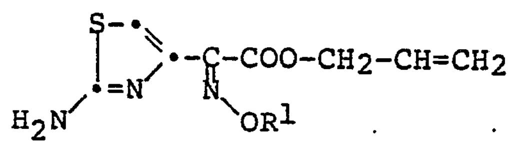 IV

worin $R^1$ niederes Alkyl, niederes Alkanoyl, niederes Alkenyl oder die Gruppe $-CH_2COOR^2$ oder $-C(CH_3)_2COOR^2$ und $R^2$ eine leicht abspaltbare Gruppe bedeuten, wobei der Ausdruck "nieder" Reste und Verbindungen mit höchstens 7 Kohlenstoffatomen bezeichnet,
dadurch gekennzeichnet, dass man eine nach Anspruch 1, 2 oder 3 erhaltene Verbindung der Formel I mit einer Verbindung der allgemeinen Formel

$$R^1\text{-}X \qquad V$$

worin X eine Abgangsgruppe bedeutet, und $R^1$ obige Bedeutung besitzt,
umsetzt.

5. Verfahren nach Anspruch 4, dadurch gekennzeichnet, dass X Halogen bedeutet.

6. Anwendung der Verfahren nach einem der Ansprüche 1 bis 5 zur Herstellung von antimikrobiell wirksamen mono-β-Lactam-, Cephalosporin- und Penicillinderivaten, deren Aminogruppe am β-Lactamring mit einer Gruppe der allgemeinen Formel

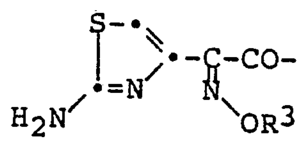 (Q)

worin $R^3$ Wasserstoff, niederes Alkyl, niederes Alkenyl oder die Gruppe $-CH_2COOR^4$ oder $-C(CH_3)_2COOR^4$ und $R^4$ Wasserstoff oder eine leicht abhydrolysierbare Gruppe bedeuten, wobei der Ausdruck "nieder" Reste und Verbindungen mit höchstens 7 Kohlenstoffatomen bezeichnet, substituiert ist.

## Claims

1. A process for the manufacture of compounds of the general formula

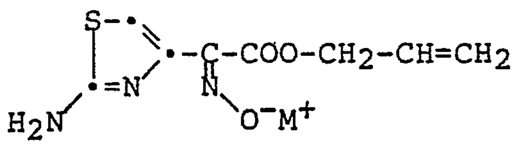

I

wherein M signifies an alkali metal atom, characterized by reacting a compound of the general formula

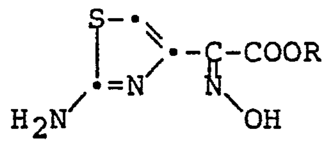

II

wherein R signifies lower alkyl with a maximum of 7 carbon atoms, with a compound of the general formula

$$CH_2=CH-CH_2-O^-M^+ \qquad III$$

wherein M has the above significance, under essentially anhydrous conditions and in a solvent consisting essentially of allyl alcohol.

2. A process according to claim 1, characterized in that M signifies a potassium atom.

3. A process according to claim 1 or 2, characterized in that R signifies ethyl.

4. A process for the manufacture of compounds of the general formula

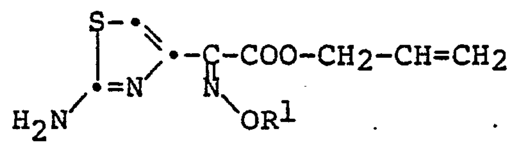

IV

wherein $R^1$ signifies lower alkyl, lower alkanoyl, lower alkenyl or the group $-CH_2COOR^2$ or $-C(CH_3)_2COOR^2$ and $R^2$ signifies a readily cleavable group, whereby the term "lower" denotes residues and compounds having a maximum of 7 carbon atoms, characterized by reacting a compound of formula I obtained according to claim 1, 2 or 3 with a compound of the general formula

$$R^1-X \qquad V$$

wherein X signifies a leaving group and $R^1$ has the above significance.

5. A process according to claim 4, characterized in that X signifies halogen.

6. The use of the process according to any one of claims 1 to 5 for the manufacture of antimicrobially-active mono-β-lactam, cephalosporin and penicillin derivatives whose amino group on the β-lactam ring is substituted with a group of the general formula

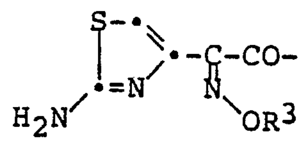

(Q)

wherein $R^3$ signifies hydrogen, lower alkyl, lower alkenyl or the group $-CH_2COOR^4$ or $-C(CH_3)_2COOR^4$ and $R^4$ signifies hydrogen or a group which is readily removable by hydrolysis, whereby the term "lower" denotes residues and compounds having a maximum of 7 carbon atoms.

## Revendications

1. Procédé de préparation de composés de formule générale

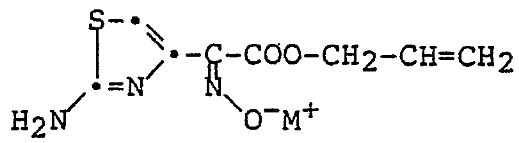 I

dans laquelle M représente un atome de métal alcalin, caractérisé en ce que l'on fait réagir un composé de formule générale

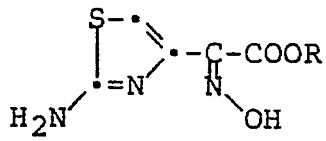 II

dans laquelle R représent un groupe alkyle inférieur à 7 atomes de carbone au maximum, en milieu essentiellement anhydre et dans un solvant consistant essentiellement en alcool allylique, avec un composé de formule générale

$$CH_2{=}CH{-}CH_2{-}O^-M^+ \qquad III$$

dans laquelle M a la signification indiquée ci-dessus.

2. Procédé selon la revendication 1, caractérisé en ce que M représente un atome de potassium.

3. Procédé selon la revendication 1 ou 2, caractérisé en ce que R représente un groupe éthyle.

4. Procédé de préparation de composés de formule générale

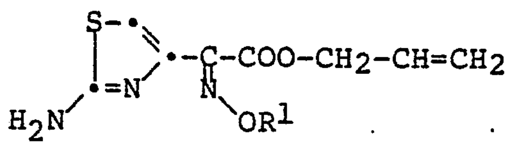 IV

dans laquelle $R^1$ représente un groupe alkyle inférieur, alcanoyle inférieur, alcényle inférieur ou le groupe $-CH_2COOR^2$ ou $-C(CH_3)_2COOR^2$ et $R^2$ représente un groupe facile à éliminer, l'expression "inférieur" s'appliquant à des groupes et composés contenant au maximum 7 atomes de carbone, caractérisé en ce que l'on fait réagir un composé de formule I obtenu selon la revendication 1, 2 ou 3, avec un composé de formule générale

$$R^1{-}X \qquad V$$

dans laquelle X représente un groupe éliminable et $R^1$ a les significations indiquées ci-dessus.

5. Procédé selon la revendication 4, caractérisé en ce que X représente un halogène.

6. Utilisation des procédés selon l'une des revendications 1 à 5 pour la préparation de dérivés de mono-bêta-lactames, de céphalosporines et de pénicillines possédant des activités antimicrobiennes et dont le groupe amino placé sur le cycle bêta-lactame est substitué par un groupe de formule générale

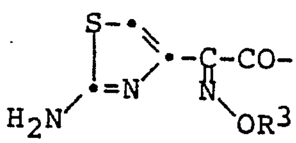 (Q)

dans laquelle $R^3$ représente l'hydrogène, un groupe alkyle inférieur, alcényle inférieur ou le groupe $-CH_2COOR^4$ ou $-C(CH_3)_2COOR^4$ et $R^4$ représente l'hydrogène ou un groupe facile à éliminer par hydrolyse, l'expression "inférieur" s'appliquant à des groupes et composés contenant au maximum 7 atomes de carbone.